# EUROPEAN PATENT APPLICATION

(11) **EP 4 137 576 A1**
(43) Date of publication of application: **22.02.2023**
(21) Application number: 21191750.5
(22) Date of filing: 17.08.2021
(51) Int. Cl.: C12N 15/74

(54) **CHROMOSOMAL INTEGRATING CASSETTE ALLOWING INDUCIBLE GENE EXPRESSION FOR PRODUCTION OF COMPOUNDS VIA FERMENTATION**

(71) Applicant: Justus-Liebig-Universität Gießen, 35390 Gießen (DE)
(72) Inventor: McIntosh, Matthew, 35398 Giessen (DE); Kretz, Jonas, 35390 Giessen (DE); Klug, Gabriele, 35415 Pohlheim (DE)
(74) Representative: Stumpf, Peter

(57) **Abstract**

The present invention provides a new system, resp. method, of genetic modification, called ACIT (Alphaproteobacteria chromosomally inserting transcription-control cassette). The invention, however, is not limited to use in Alphaproteobacteria. The modification is implemented within the bacterial chromosome and therefore permanently implemented within the microorganism.

## Description

Microbial fermentation processes are important methods for producing chemical compounds in a sustainable way, because their feedstock consist of agriculture-based raw materials and they are usually less energy-consuming than classical processes due to the low fermentation temperatures in comparison with classical chemical reactions.

### Field of the invention

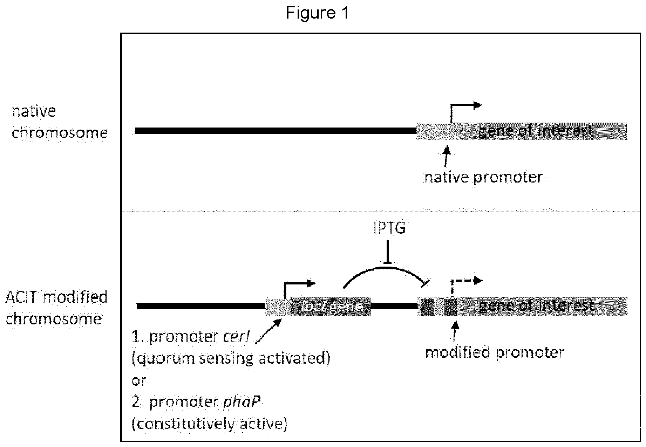

The invention relates to the field of production of substances or mixtures of substances, e.g. curdlan, polyhydroxybutyrate (PHB) or galactoglucan, by use of inducible gene expression. The invention is designed to modify bacterial chromosomes in order to achieve tight control over inducible gene expression (see Figure 1).

### Background of the technology

Regulation of gene expression is essential for survival. The mere presence of genes (i.e., the genome) does not ensure survival. Rather, each gene needs to be expressed appropriately, at the right rate and the right timing, according to its role in the survival of the organism. Many, if not most, of the regulatory mechanisms that control gene expression throughout nature remain undiscovered. Manipulation of gene expression holds immense potential for taking advantage of the impressive abilities of life. For example, modified bacterial pathogens could be used in medicine to kill genetically related bacteria through the control of gene expression. Artificial control over gene expression is also useful in bacterial cell factories for the production of antibiotics and useful vitamins, proteins and polysaccharides and renewable energy sources. Manipulation of gene expression is a favourite strategy in biology for the study of gene regulatory circuitry necessary for life. It is useful for detecting and characterizing novel regulatory mechanisms. Current state of the art research offers a number of possibilities for manipulating gene expression, using a range of physical and chemical inducers, each involving advantages and disadvantages. However, the problems involved with achieving stable and controllable gene expression are many.

Inducible gene expression is currently (within the current state of the art) applied using inducible expression systems carried on plasmids. A plasmid is an extrachromosomal DNA molecule within a cell that can replicate independently of the chromosome. There are at least four major problems with inducing gene expression using plasmids. Firstly, plasmids can be lost during cell reproduction, particularly when they create a burden on cellular metabolism. Prevention of plasmid loss can be minimized by the use of selective criteria, such as the inclusion of antibiotics in the growth medium. However, these tend to have diverse negative side effects on cell growth and other bacterial behaviours. Secondly, most replicating plasmids exist at 5-100 copies per cell, which is suitable for inducible overexpression but not repression of expression. Multiple copies of the inducible expression system mean that leaky or unwanted expression will always be present. Thirdly, whenever the inducible expression of several genes that are organized in an operon (an assembly of co-transcribed genes) is desired, this requires the laborious cloning of these genes into a plasmid, a task that can consume considerable time and resources. Fourthly, a plasmid is unable to control the expression of the genes of interest while located at their natural site in the chromosome. The most common solution to this problem to date is to first delete the gene of interest from the bacterial chromosome (to create a mutant), and then to reintroduce the gene of interest under the control of an inducible gene expression system carried on a plasmid. However, this solution does not avoid the leaky expression problem associated with multiple copy numbers of the plasmid. It also requires considerable efforts to generate the mutant as well as cloning the gene of interest. Furthermore, it creates an unstable genome (i.e., upon addition of an extra-chromosomal plasmid) that must be maintained using antibiotics.

Document US20170002363A1 reveals a system for genetic modification including the application of the EiL-System for modulating the degree of genetic expression. The Eil system consists of the *eilR* gene and the promoter controlled by the EilR protein that activates expression of the *lacI* gene. The EiL-System ensures the control over the /ac/-system by usage of crystal violet as inhibitor of gene expression. The system described in US20170002363A1 cannot be implemented chromosomically, i.e. within chromosomes; it is confined to be implemented within separate plasmid-structures, to be inserted additionally into the microbial cells.

The current state of the art, i.e. inserting the desired genetic modification to the microbial cells via additional plasmid-structures is prone to a gradual loss of the genetic information, so that "refreshing" is necessary after several generations of microbial growth.

### Content of the invention

The objective of this invention is two-fold: the design of a genetic system capable of tight control of expression of a gene of interest, and the design of a genetic system capable of being adjusted and adapted to operate in any bacterium.

In order to achieve the above objective, the present invention provides a new system, resp. method, of genetic modification, called hereinafter ACIT (Alphaproteobacteria chromosomally inserting transcription-control cassette). The invention, however, is not limited to use in Alphaproteobacteria. ACIT is operatively linked to the gene of interest as is described below, allowing the control of the gene of interest.

ACIT solves the problems described above by using a plasmid that integrates into the host chromosome of the bacterial host. Thus the loss of plasmids during cell reproduction is avoided; the possibility is provided to implement not only overexpression, but also repression of expression in order to fine-tune the expression; if inducible expression of several genes that are organized in an operon is desired, it is no longer necessary to perform the laborious cloning of these genes into a plasmid; controlling the expression of the genes of interest while being located at their natural sites in the chromosome is possible.

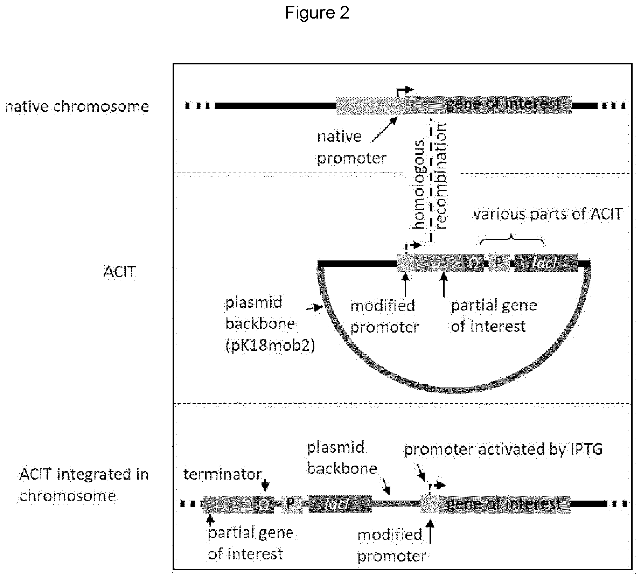

The integration into the chromosome of the bacterial host is specific, targeting the native site of the gene of interest via homologous recombination (see Figure 2). There are several striking advantages of using an integrated plasmid. Firstly, integrated plasmids are more stable than self-replicating plasmids, since these are far less likely to be lost than replicating plasmids, particularly when they create a burden on cellular metabolism. Hence, antibiotics are not necessary for their maintenance. Secondly, the use of an integrating plasmid in ACIT provides for a tighter control over gene expression. While most replicating plasmids exist at 5-100 copies per cell, integrated plasmids adopt the copy number of a chromosome, i.e., 1-2 copies per cell. This allows the controlled transcription depletion or silencing of the gene of interest without disruption of gene sequence. Thus, a tremendous advantage of ACIT is the control over a single copy of the gene of interest. Thirdly, ACIT can control the expression of an operon of genes in their native location on the chromosome without altering their physical location on the chromosome and without the need to clone the entire operon into an extrachromosomal plasmid.

ACIT solves further major problems in the field of inducible expression, e.g.:
Within microbiology, most of the information on available inducer system comes from the gammaproteobacteria, such as *Escherichia coli,* or from Streptomyces such as *Bacillus subtilis.* Less is known about inducible systems outside of these model bacteria. Moreover, application of inducible systems from model bacteria in lesser-studied bacteria is fraught with problems. For example, the alphaproteobacterium *Sinorhizobium meliloti* is an agriculturally important soil bacterium because of its ability to fix atmospheric nitrogen in symbiotic association with alfalfa (*Medicago sativa*). An arabinose-inducible system, which works well in E. *coli,* does not function in S. *meliloti* for unknown reasons. Thus, the arabinose-inducible system cannot be used in S. *meliloti.* This example underscores the importance of developing inducible expression systems that are useful in a broad range of bacteria. ACIT solves this problem since its design allows for adjustment and modifying it to make it suitable for use in any bacterium, including both model and non-model bacteria as well as less-studied model bacteria.

The method of genetic modification via ACIT is exemplarily described by a process for controlling cell growth and cell behavior and for producing exopolysaccharides and other compounds of interest.

The invention consists of six specifically arranged nucleotide sequences, referred to in this document as parts. Specific to this invention is (a) the order of the six parts, which is essential for the functioning of ACIT and (b) the nucleotide sequence of part one. The benefit of ACIT is to achieve an extremely efficient control over the transcription rate of a chromosomally located gene of interest through the addition of chemical inducers. Isopropyl-β-D-thiogalactopyranoside (IPTG) is applied here for fine-tuning the transcription activation. Upon making the appropriate adjustments to ACIT, other chemicals such as crystal violet may serve as the chemical inducer of tight transcription repression.

Part one is a promoter sequence flanked by Lac operator sites. This nucleotide sequence is novel and is the most important part in this invention, since it provides the ability to control the expression of a specifically chosen gene of interest using IPTG as the chemical inducer of gene expression.

Part two is the *lacl* gene coding for the Lacl repressor protein.

Part three is a nucleotide promotor sequence which is independently chosen from the list of nucleotide promotor sequences comprising the nucleotide promotor sequences Pcerl, PphaP, Pvan. This part determines the expression level of the *lacl* gene. Exemplarily the sequence of the *cerI* promotor, flanked by SalI (GTCGAC) and Sacl (GAGCTC) is provided as SEQ ID no. 6.

Part four is a transcription terminator.

Part five is the publically available cloning vector pK18mob2 which provides two important properties: kanamycin resistance and the inability to replicate in hosts other than *Escherichia coli* and some closely related gammaproteobacteria. These two features ensure that ACIT acts as a suicide plasmid (in bacteria other than E. *coli* and its close kin), integrating into the host chromosome. If desired, ACIT could also be used in E. *coli* if part five (pK18mob2) is replaced with another vector which acts as a suicide plasmid in *E*. *coli.*

Part six is a specific sequence from the host chromosome, which ensures homologous recombination between ACIT and the host chromosome and thus integration of ACIT at the chromosomally located site of a specific gene of interest.

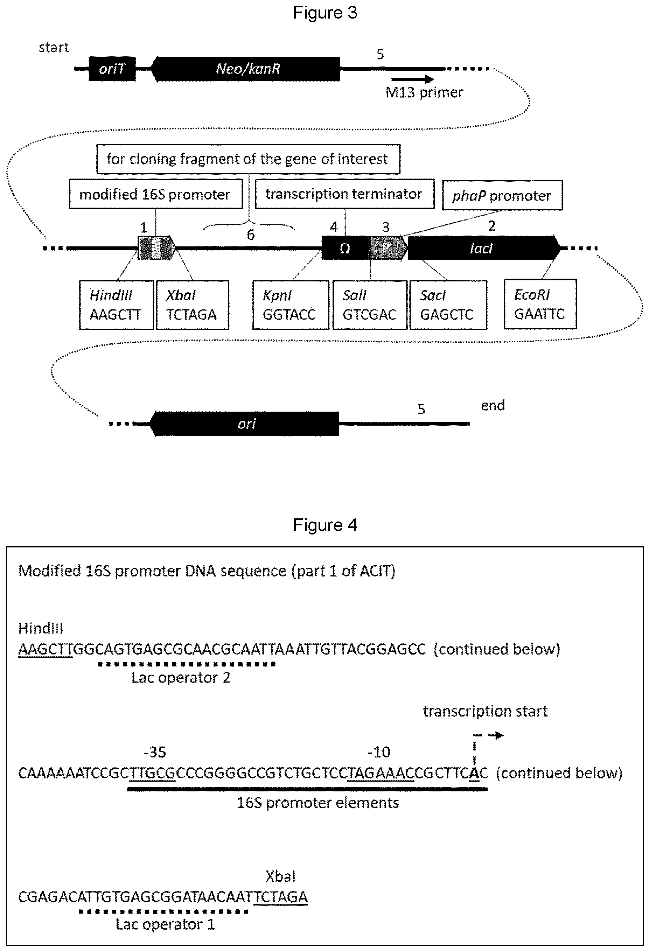

Following, these six parts are described in more detail (see Figure 3 for a plasmid map of the parts):
1. Indigenous promoter flanked by Lac operators (120 bp, see Figure 4 for nucleotide sequence)
2. lacI gene (1107 bp)
3. variable nucleotide promotor (variable size)
4. transcription terminator (238 bp)
5. suicide (integrating) plasmid conferring antibiotic resistance (2984 bp)
6. fragment from gene of interest for homologous recombination with chromosomal (300-1000 bp)

### Part 1. Indigenous promoter flanked by Lac operators

The *lac* promoter from *E*. *coli* is one most widely used inducible systems in microbiology. However, in many bacteria, the *lac* promoter is too weak, or too leaky, or both. The solution to this problem is to insert an active promoter between two Lac operators, using the Lac promoter as a model, resp. example herein. It is important that the promotor applied is functioning within the host organism (i. e. being active) and is controlled by the two Lac operators flanking it. The maximum length of the promoter, including the flanking Lac operators, is 150 - 200 bp.

The two Lac operator sequences are: GGCAGTGAGCGCAACGCAATT (SEQ ID no. 1) and ATTGTGAGCGGATAACAATT (SEQ ID no. 2). These are indicated in Figure 4. According to the Lac model, the number of nucleotides between these two operators must be 72 bp. Within the 72 bp should be the DNA sequence of a promoter, whose activity is preferably constitutive and strong. In this version of ACIT, the promoter is from the 16S gene (RSP_4352) of *Rhodobacter sphaeroides,* previously shown to be a strong promoter whose activity correlates well with bacterial growth. The promoter is flanked by the Lac operator sites given above. The 16S promoter shows no activity when the Lac operators are bound to LacI. The lack of activity is because of the interaction between Lacl and the two Lac operators, forming a loop in the DNA which excludes any interaction between the RNA polymerase and the promoter, thereby repressing transcription. In the absence of Lacl or when Lacl is sequestered by IPTG, the DNA is free from structural hindrance to the RNA polymerase, and the promoter shows typical strong and constitutive activity. It is obvious to the person of ordinary skill in the art that the exemplary usage of the *lac* promoter from *E*. *coli* is not meant to confine the scope of the invention; any other promoter, providing the same features, may be used instead without leaving the scope of the invention.

### Part 2. lacI gene

The *lacl* gene exemplarily used herein is derived from *E*. *coli* and codes for the Lacl repressor, a DNA-binding protein that inhibits transcription initiation. The protein contains a helix-turn-helix domain, which specifically binds to an 18 bp DNA sequence known as the Lac operator. Strength of transcription repression by Lacl depends upon the interaction between the protein and the DNA, and this is in turn influenced by the abundance of Lacl and the presence of lactose or IPTG. Lactose and IPTG disrupt the binding between Lacl and its operator. It is obvious to the person of ordinary skill in the art that the exemplary usage of the *lacl* gene from E. *coli* is not meant to confine the scope of the invention; any other gene coding for a DNA-binding protein, providing the same features, may be used instead without leaving the scope of the invention.

### Part 3. nucleotide promotor

Figure 1 schematically shows that within ACIT several promotor systems may be applied, indicating the great variability/applicability of the ACIT system - here exemplarily shown by either the promoter for *cerI* (when used in *R. sphaeroides*) or the promoter for *phaP* when used in other bacteria.

The promotors exemplarily shown in Figure 1 are not meant to be restrictive to the scope of the invention. Any other promotor may be used, e.g. Pvan (VanR), where vanilic acid is the compound used for induction. Indeed, the possibility to use different promotors within ACIT is one of the core features of ACIT, since part of the design of ACIT is the use of specific DNA restriction digest sites (see Figure 3 for locations of the DNA restriction sites), resp. specific DNA restriction cleavage sites, which allow the replacement of any part without disturbing any of the other parts. The expressions "digest sites" and "cleavage sites" are used herein synonymously indicating sites of precise cleavage of the DNA sequence without resulting in further decay of the DNA sequence. Hence, in each case, replacing a complete promotor sequence can be performed in a one-step cloning process.

### Promoter of cerI (PcerI)

The first promoter exemplarily described herein is the *cerI* promoter, referred to as Pcerl. In its native location in the chromosome of *Rhodobacter sphaeroides,* it controls the expression of CerI, the synthase for a quorum sensing autoinducer molecule. This promoter has been shown to be dependent upon the presence of autoin-ducers (positive autoregulation). The two important features of this promoter for its use in ACIT are its high activity and that this activity occurs very early in the growth phase. These two conditions mean that the promoter of *cerI* is suited for the early, strong expression of LacI, which in turn, provides a nice control over the modified promoter and thus the expression of the gene of interest.

### Promoter of phaP (PphaP)

The second exemplary variant of the ACIT system uses the promoter of *phaP.* The reason that this promoter was exemplarily used is because Pcerl does not function in any bacterium other than *R. sphaeroides,* due to its dependency upon both the autoinducer molecules and CerR, the autoinducer receptor protein. Therefore, PphaP was used in ACIT constructs designed for bacteria other than R. *sphaeroides,* since PphaP was found to be active in a range of different bacteria. PhaP is a phasin, a protein which binds to polyhydroxybutyrate (PHB) which accumulates as a carbon storage granule in many microbes. In these other bacteria, PphaP activity was constitutive, from early in the growth phase to stationary phase. This provided strong expression of Lacl and thus good control over the expression of the gene of interest.

### Part 4. transcription terminator

Upon integration of ACIT into the host chromosome, it is important that a strong transcription terminator be included to silence the native promoter controlling transcription of the gene of interest. To ensure this, ACIT carries the *rpoClthrA* tandem terminator, derived from E. *coli.* It is obvious to the person of ordinary skill in the art that the exemplary usage of the *rpoClthrA* tandem terminator, derived from E. *coli,* is not meant to confine the scope of the invention; any other terminator, providing the same features, may be used instead without leaving the scope of the invention.

### Part 5. suicide plasmid pK18mob2

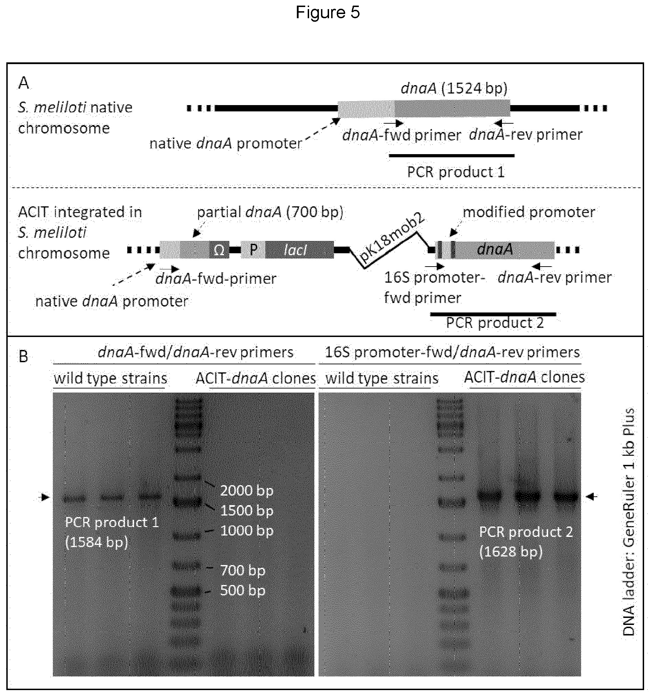

The essential components of an integrating plasmid are that it carries an antibiotic resistance marker and a replicating region which allows self-replication in E. *coli* (to allow conjugation) but not in the bacterium of interest. The plasmid pK18mob2 has frequently been used as an integrating (also known as suicide plasmid) vector suitable for research on a wide range of bacteria, including alphaproteobacteria and Gram positive bacteria. It carries the pMB1 replicon, meaning that self-replication is restricted to E. *coli* and closely related species of the genera *Salmonella* and *Serratia.* Also importantly for the ACIT construct, it supplies a multiple cloning site, a *lacZ* alpha fragment and sites for M13 primers for sequencing. Upon insertion into the chromosome, a selection of primers can be used to confirm the insertion via PCR (see Figure 5). Finally, pK18mob2 carries a kanamycin resistance cassette, allowing for the selection of homologous recombination events. It is obvious to the person of ordinary skill in the art that the exemplary usage of the plasmid pK18mob2 is not meant to confine the scope of the invention; any other plasmid, providing the same features, may be used instead without leaving the scope of the invention.

### Part 6. fragment from gene of interest for homologous recombination with chromosome

Integration into the chromosome simultaneously silences the indigenous regulatory control over that gene and replaces it with the inducible system. To ensure that ACIT integrates into the chromosome at a specific location (i.e., directly upstream of the gene of interest), a fragment of 300-1000 bp that is identical to the gene of interest (allowing homologous recombination) must be present within ACIT. Usually, the fragment will begin upstream of the native ribosome-binding site of the gene of interest and stop somewhere within the coding region. For most bacteria, a region of at least 300 bp (up to 1000 bp) is sufficient to ensure homologous recombination.

Figure 2 provides a detailed overview of ACIT, including the location of each of the parts before and following integration into the chromosome.

Natural gene regulation can be highly complex, presenting enormous challenges to the goal of achieving an appropriate level of control over gene expression. Many genetic components function poorly or not at all when transferred to non-host bacteria, meaning that these parts need to be tested and perhaps modified or even replaced in order to achieve the necessary level of control.

Gene expression can vary greatly from gene to gene. Some genes are constitutively expressed, others are regulated according to environmental factors (e.g., nutrient availability) or synchronised according to cell division, population density and growth phase. Also varying greatly from gene to gene is the strength of expression. Examples of strong gene expression are the *rrn* genes, which produce the rRNA component of the ribosomes. In E. *coli,* their expression activity is relatively strong, peaking during early exponential growth. Thereafter, their activity drops and is virtually silent upon entry into stationary phase. Regulation of the *rrn* gene expression and its impact on bacterial physiology remains mostly uncharacterized in E. *coli,* despite more than 50 years of research. This is likely due to a number of reasons. Firstly, each bacterial genome has several copies of the *rrn* genes. Secondly, the *rrn* genes are essential, meaning that they cannot be deleted from the genome. Thirdly, there is a lack of appropriate inducible expression systems capable of manipulating *rrn* gene expression. Even less is known about the regulation of *rrn* gene expression in bacteria other than *E*. *coli,* since there are currently no options for either gene deletion or temporarily silencing their expression.

Another example is the *relA* gene, responsible for the production and degradation of the hunger alarmone ppGpp. Expression of the native *relA* is very weak in *S*. *meliloti.* The problem arises when the leaky *relA* expression from a fully repressed Lac system carried on a plasmid is several times stronger than the native expression levels. This setup cannot avoid an over-expression of *relA.* This means that using the Lac system, *relA* can be over-expressed, but not depleted or even expressed at normal levels. The inability to deplete *relA* leaves only one option open to researchers interested in understanding the role of *relA* in the cell. That option is to delete or disrupt the *relA* gene. However, such *relA* mutants grow poorly and are plagued with the rapid appearance of secondary mutants, meaning that the *relA* mutant genotype is unstable. The ability to temporarily silence *relA* expression via tight artificial repression provides a solution to this problem, allowing less time for problematic secondary mutations to arise.

These two examples provide a glimpse of the challenges facing researchers attempting to study the regulation of gene expression. In each case, the conventional approach of gene deletion or disruption is unsatisfactory. In contrast, the use of ACIT, which allows the option of temporary gene silencing or depletion of gene expression, would allow for further research on these two important areas of research.

A third category of genes that are difficult to artificially regulate are the essential genes, meaning that functional loss of such genes results in cell death. Hence, temporary silencing or depletion of gene expression is the only way to study such genes. The gene *dnaA* is essential for controlling chromosome replication in almost every bacterial species. Artificial control over *dnaA* expression via the use of inducers means that chromosome replication, and thus growth, can be controlled by supplying the inducers at specific concentrations.

### Trouble shooting and solutions using the ACIT design

Sometimes the gene of interest will be located in an operon of genes that are all co-expressed. ACIT offers a large advantage by replacing the native promoter and allowing the controlled expression of the operon. The problem arises, however, when the goal is to specifically control the expression of only one of those genes within the operon. The solution will be to delete the gene of interest, and integrate it along with ACIT at another location in the chromosome, requiring an additional step.

Sometimes the gene of interest is extremely small. A minimum of 300 bp is needed for the homologous recombination with ACIT. One solution is to use a plasmid encoded ACIT, although this can only ensure over-expression, not depletion of gene of interest. Another solution is first to delete the small gene of interest from the chromosome and then integrate it back into the chromosome under the control of ACIT.

Sometimes it is desirable to control multiple genes simultaneously at their native locations in the chromosome. Suicide integration only allows one copy of ACIT per genome. A solution is to excise ACIT (via homologous recombination, e.g., using the negative selection of *sacB* and a second homologous region) leaving only part 1 upstream of the gene of interest. This would mean that ACIT could then be modified at part 6 and re-integrated into the chromosome at the native site of the second gene of interest.

The experimental application of ACIT to control gene expression in two different types of microorganisms, *R. sphaeroides* and S. *meliloti* exemplarily revealed that with ACIT extensive control over the expression of numerous genes of interest is possible. ACIT provides tight, finely tuned control over the expression of the gene of interest, or - exemplarily - in the case of *mraZ,* the gene cluster of interest. In particular, ACIT control over gene expression is very tight in S. *meliloti,* providing excellent, non-leaky control over *sinR* and *expR* expression. Moreover, the modifications reveal the flexibility and ease of adaptability of ACIT. Thus, the examples shown are not confining the scope of the invention. It is especially advantageous introducing the ACIT system into microorganisms which already naturally produce the product of the gene of interest, for they are adapted to the product of the gene of interest so that its production does not negatively interfer with the viability of the microorganisms, thus providing much better yields within the fermentation process.

Exemplarily, the complete sequence of the ACIT-System is shown as SEQ ID no. 5, the fragment of the gene of interest in this example being a part of the sequence of the gene coding for PhaC, the synthase of PHB (polyhydroxybutyrate) in *Rhodobacter sphaeroides.*

Further examples of applicable fragments of genes of interest are (cf. also table 1, below):
- Fragments of the sequence of SinR, a transcription activator which is essential for the activity of the promoter of *sinl,* the gene coding for the AHL (quorum sensing inducer) synthase of *Sinorhizobium meliloti* (e.g. SEQ ID no. 12),
- Fragments of the sequence of ExpR, an AHL receptor protein of *Sinorhizobium meliloti,* which controls transcription in response to binding of AHLs (e. g. SEQ ID no. 13),
- Fragments of the sequence of PhaC, the synthase of PHB (polyhydroxybutyrate) in *Rhodobacter sphaeroides* (e.g. SEQ ID no. 14),
- Fragments of the sequence of *mraZ,* the first gene in the *dcw* gene cluster, which controls cell division in *Rhodobacter sphaeroides* (e.g. SEQ ID no. 15),

Whenever hereinafter a range of values, for example regarding numbers of base pairs of nucleotides (bp), times etc. is mentioned, always additional sub-ranges within the mentioned ranges are contemplated and are within the present disclosure, even if not explicitely mentioned, as is known to and recognized by the person of ordinary skill in the art.

### Detailed embodiments of the invention

For exemplarily testing the inventive method of genetic modification (ACIT), two bacteria were used: *R. sphaeroides* and S. *meliloti.* In *R. sphaeroides,* two genes were selected as genes of interest: *phaC* (RSP_0832), which is the synthase for polyhydroxybutyrate (PHB), and *mraZ* and the *dcw* gene cluster that control cell division. In the case of *phaC,* the removal *of phaC* from its promoter (i.e., promoterless *phaC)* is known to almost completely remove PHB production. In the case of the *dcw* gene cluster, the promoter of *mraZ* is according to the state of the art considered to control the expression of *mraZ* as well as the entire *dcw* gene cluster. Upon insertion of the exemplary nucleic acid sequences derived from the genes of interest *(phaC* and *mraZ)* into ACIT, forming the inventive method of genetic modification, and the integration of ACIT at each of the two chromosomal locations, *phaC* and *dcw* expression were under the control of the promoter of the inventive method of genetic modification and IPTG levels.

Two additional genes were also selected for exemplarily testing the inventive method of genetic modification in S. *meliloti: sinR* and *expR.* SinR is the major transcription activator for the expression of Sinl, the acyl homoserine lactone (AHL) synthase. ExpR is, like SinR, a transcription regulator. Unlike SinR, however, ExpR functions as the major AHL receptor, and the ExpR-AHL complex is the major activator of the *wge* genes that are essential for production of the exopolysaccharide galactoglucan. Importantly, the ACIT control over the expression of all four genes, *phaC, mraZ, sinR* and *expR* is measureable. Below, results are provided which demonstrate ACIT control in terms of either downstream gene expression (e.g., a Sinl::mCherry fusion provides an indication *of sinR* expression) or downstream secondary metabolites (e.g., PHB *for phaC* or galactoglucan for *expR)* or a growth phenotype (e.g., *mraZ* and the *dcw* gene cluster).

Additionally to the two examples discussed above, a third example is presented herein, the application of ACIT for the production of curdlan. Regarding the production of curdlan via biosynthesis, the expression of three genes is necessary. Their position within the genetic map of *Agrobacterium tumefaciens* is well known to the person skilled in the art, for it is already published (e. g. *Agrobacterium sp.* CGMCC 11546, chromosome 2).

**Table 1: Summary of exemplary genes of interest and their associated phenotypes**

| Gene of interest | Bacterium | Phenotype |
|---|---|---|
| *phaC* | *R. sphaeroides* | PHB accumulation |
| *mraZ* | *R. sphaeroides* | cell shape |
| *sinR* | *S. meliloti* | expression of Sinl::mCherry |
| *expR* | *S. meliloti* | expression of *wgeA:* cerulean/ galactoglucan production |
| *crdA, crdS, crdC* | *A. tumefaciens* | biosynthesis of curdlan |

### Measurement of ACIT performance

### phaC

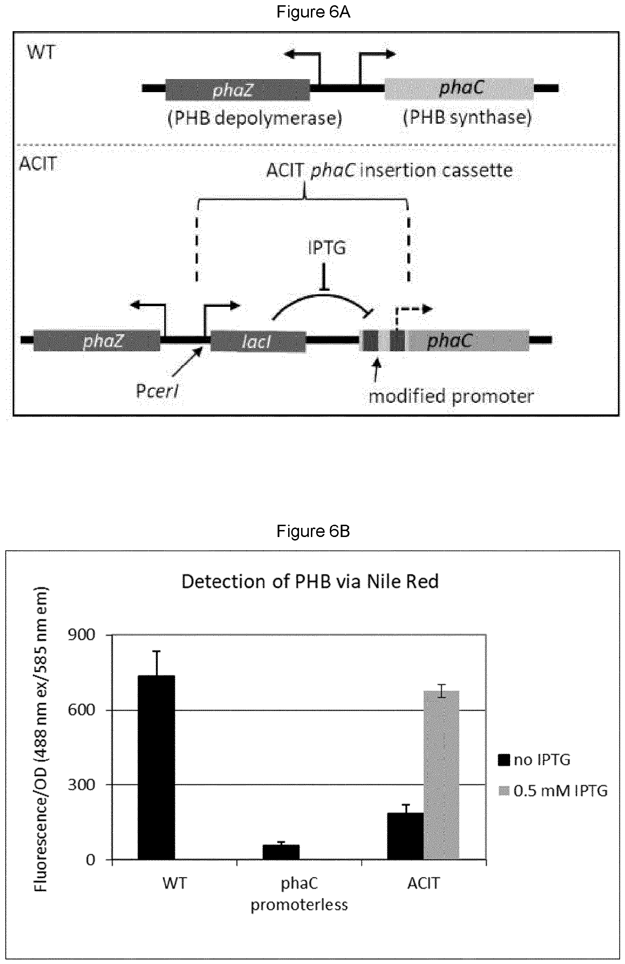

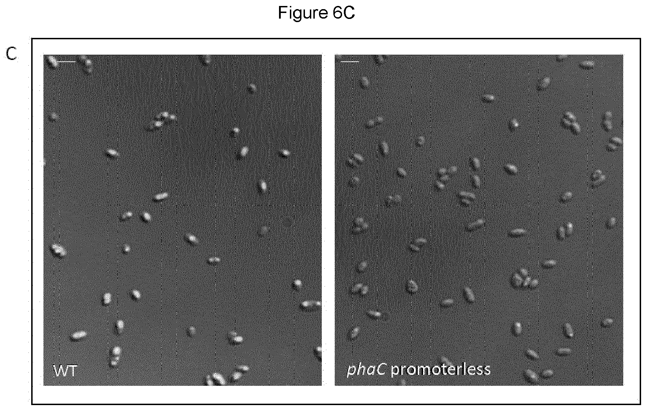

PHB is a carbon-rich storage molecule located in the bacterial cytoplasm. When cells containing PHB are stained with Nile Red, fluorescence can be detected. The gene *phaC* codes for the PHB synthase. In the presence of IPTG, *phaC* expression results in PHB production, while in the absence of IPTG, a lack or very weak *phaC* expression results in very low levels of PHB production. Figure 6A-C show exemplary results of ACIT used to control expression of *phaC* and PHB accumulation in *R. sphaeroides.* Fluorescence detected from the complex between PHB and Nile Red was detected using a fluorescence reader (Figure 6B) and fluorescence microscopy (Figure 6C). Cells with PHB granules appear brighter (Figure 6C, left) than those lacking PHB granules (Figure 6C, right).

### mraZ/dcw

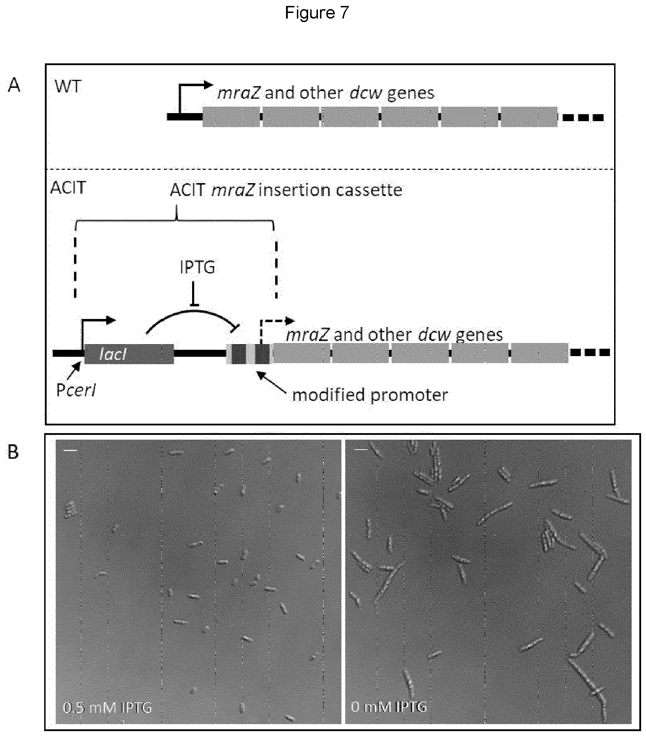

*mraZ* is the first gene of the *dcw* gene cluster controlling cell division and cell wall synthesis. Bacterial *dcw* gene clusters are highly conserved among bacteria. Their arrangement suggests that these are expressed as a single transcriptional unit. In *R. sphaeroides,* the *dcw* gene cluster consists of 20 genes. The fact that the promoter of *mraZ* also controls the expression of the entire *dcw* gene cluster means that the investigation of the importance of these genes for cell growth, and how these are regulated, is extremely difficult using conventional genetic deletion-and-complementation procedures. The large size of the cluster makes their cloning almost impossible. Furthermore, the fact that they are essential for growth means that they cannot be deleted. ACIT solves this problem by controlling their expression via the insertion of the IPTG inducible promoter in the chromosome. Figure 7 exemplarily shows the usage of ACIT to control expression of the *dcw* gene cluster and cell shape in *R. sphaeroides.* In the absence of IPTG and *dcw* gene expression, cells are defective in cell division and appear up to 10 times longer than cells with IPTG (Figure 3B).

### sinR

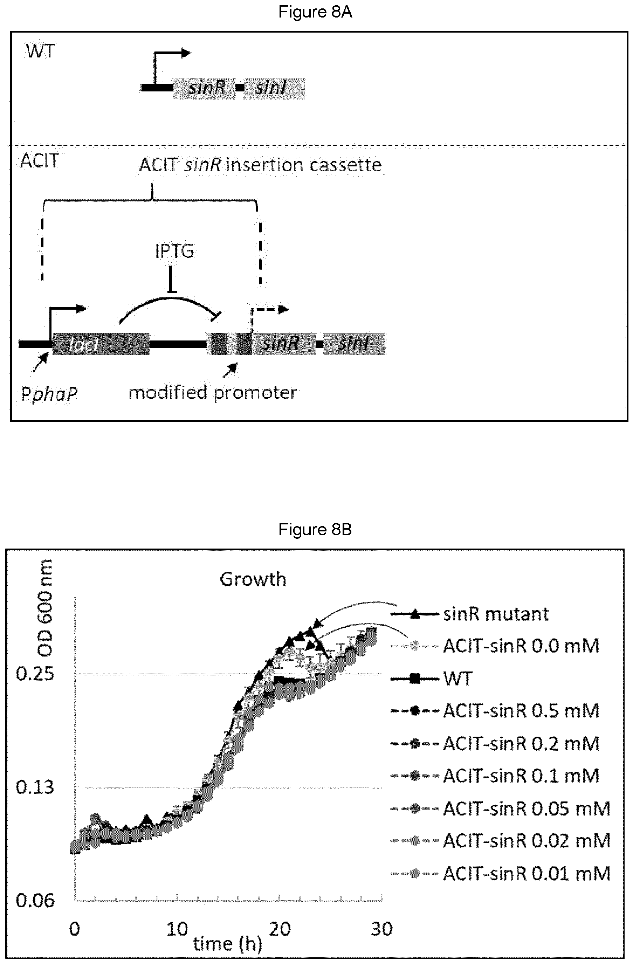

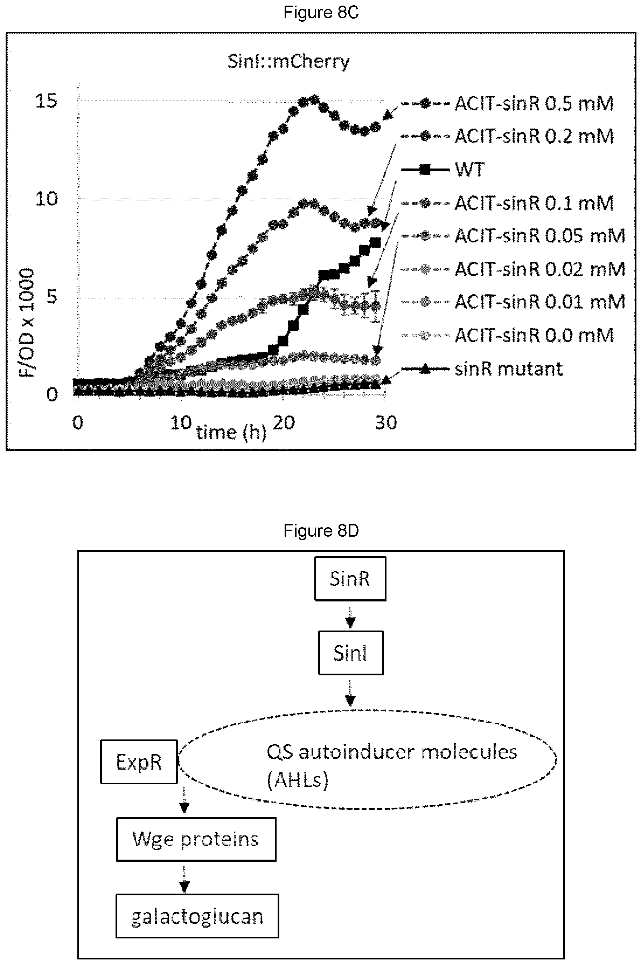

Quorum sensing in S. *meliloti* depends upon the presence of acyl homoserine lactones (AHLs) as signalling molecules, which provide an indication of population density. The synthesis of AHLs depends upon Sinl, the AHL synthase. The expression of Sinl depends upon SinR, the major transcription regulator of *sinI.* Replacement of the native *sinR* promoter with ACIT allows for the control over downstream *sinl* expression. Figure 8A-C exemplarily shows ACIT being used to control expression of *sinR* and the downstream control over *sinl* expression in S. *meliloti.* In the absence of IPTG, *sinl* expression is undetectable. Increasing levels of IPTG results in *sinR* expression and a corresponding increase in *sinl* expression.

### expR

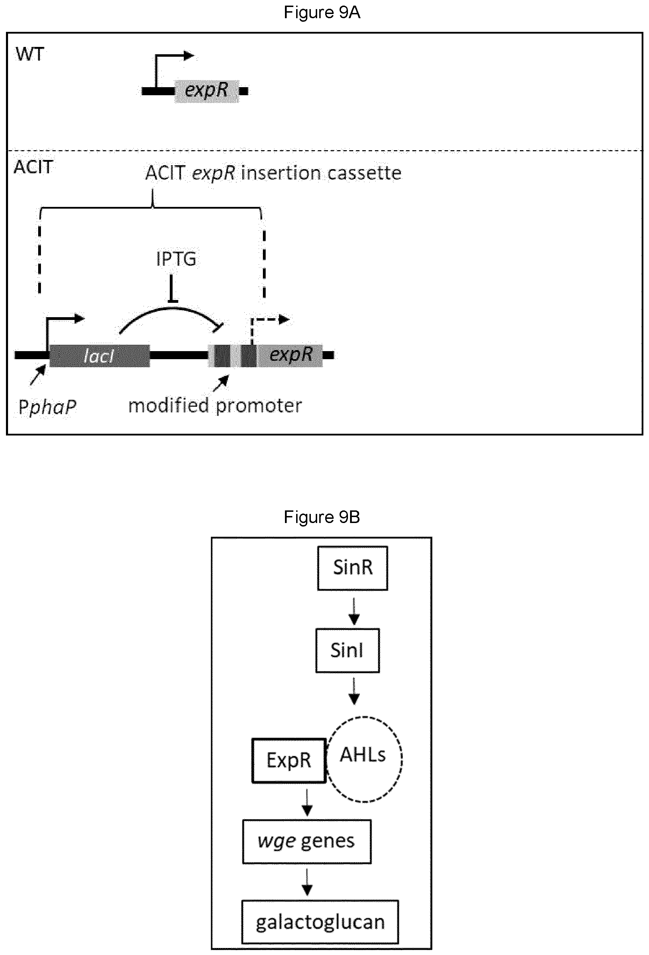

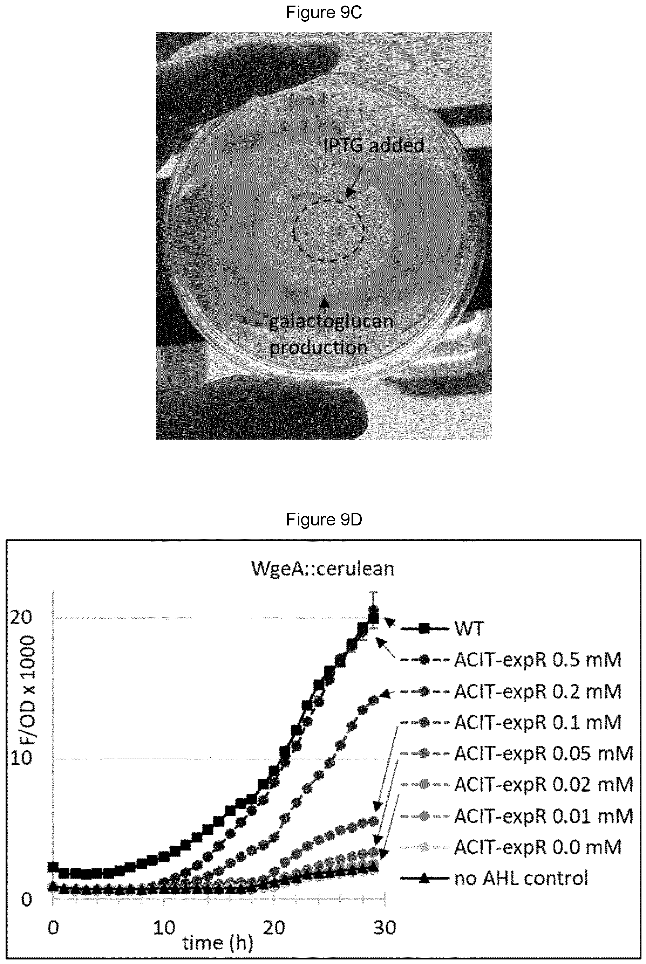

The receptor for AHLs in S. *meliloti* is ExpR. When the AHL-ExpR complex forms, the ExpR component binds to at least 30 different locations on the chromosome. This binding action either represses or activates the target genes. One of the targets of the AHL-ExpR complex is the *wge* gene cluster that is essential for the synthesis of galactoglucan. Galactoglucan is one of the major exopolysaccarides produced by S. *meliloti,* and is important in biofilm formation, mobility and symbiosis with legume plants. It is also strongly activated by quorum sensing through the AHL-ExpR complex. Figure 9A-D exemplarily show ACIT being used to control expression of *expR* and galactoglucan production in S. *meliloti.* In the absence of IPTG, *wge* expression is undetectable. Increasing levels of IPTG results the expression of *expR* and a corresponding increase in *wge* expression.

The description of the whole *wge* gene cluster, necessary for the galactoglucan production via biosynthesis can be found in the publication of A. Becker et al., "The 32-kilobase exp gene cluster of Rhizobium meliloti directing the biosynthesis of galactoglucan: genetic organization and properties of the encoded gene products.", Journal of Bacteriology, 1997, 179(4): 1375-1384. The coordinates of the *wge* genes in the genomic map can be found on the NCBI web site which provides the genetic map, the *wge* genes are being located from position 968095 to 997228 on the megaplasmid pSymB of *Sinorhizobium meliloti.*

### Example of chromosomal integration of ACIT at the essential gene dnaA

In order to prepare ACIT for integration into the host chromosome at the native *dnaA* locus, a 700 bp fragment which included the translation start plus the following 697 bp was inserted into ACIT. This fragment formed part 6 of ACIT (see Figure 3 for location of part 6). This fragment was inserted via the restriction enzyme sites Xbal and Kpnl. Sequencing of the modified ACIT (i.e., upon insertion of the fragment) was performed via the standard primer M13 (GCTGCAAGGCGATTAAGTTG, (SEQ ID no. 3)), providing a sequence of parts 1 and 6 (see Figure 3 for location and orientation of M13 (5'->3')).

Integration of the construct into host chromosome was performed by a simple biparental conjugation between the E. *coli* strain S17-1 and the bacterium of study, Si*norhizobium meliloti* Rm2011. For the conjugation, S17-1 and S. *meliloti* were harvested from fresh agar cultures and resuspended and mixed at a ratio of 1:1. The mixture was then dropped on rich medium agar in 30 µL spots and incubated at 30°C for 1-6 days. In some cases, depending upon the gene of interest, the presence of IPTG during conjugation may be helpful to ensure gene expression (in the case of essential genes) upon integration of ACIT into the chromosome. Following the incubation, the growth spot containing the bacterial mixture was resuspended in liquid medium and plated on complex agar supplemented with kanamycin (to select for ACIT) and spectinomycin (to select for S. *meliloti).* Spectinomycin is important because it prevents growth of S17-1. Alternatively, some other condition such as minimal salts agar with does not support the growth of S17-1 could be used. Single colonies should appear within a week of incubation.

Confirmation of integration of ACIT at the chromosomal site of the gene of interest was performed by using colony PCR. Ideally, for the colony PCR, one primer should be specific to ACIT and a second primer specific to the chromosome. See Figure 5 for map of ACIT along with location of primers.

Exemplary description of the transformation of a host (a host bacterium) with ACIT:
Transformation of the host bacterium with ACIT is performed by a simple biparental conjugation between the donator bacterium, e.g. *Escherichia coli* strain S17-1, and the host bacterium, e.g. *Sinorhizobium meliloti* Rm2011. First, as a preparative step, ACIT is used to transform E. *coli,* either by calcium competent E. *coli,* or by electro-poration of *E*. *coli* using a common method readily available in the published literature. Then, for conjugation, *E*. *coli* and S. *meliloti* are harvested from separate, fresh agar cultures and resuspended in fresh liquid medium and mixed at a ratio of 1:1. The mixture is then pipetted onto rich medium agar (with yeast extract) in 30 µL spots and incubated at 30°C for 1-2 days. Conjugation occurs during this growth period. In some cases, depending upon the gene of interest, the presence of IPTG during conjugation may be helpful to ensure gene expression (in the case of essential genes) upon integration of ACIT into the chromosome. Following the incubation, the spot containing the bacterial mixture on agar is resuspended in 0.5 mL of liquid medium. About 30-50 µL of the resuspension is plated on growth agar supplemented with kanamycin (to select for ACIT) and spectinomycin (to select for *S*. *meliloti).* Spectinomycin is important because it prevents growth of E. *coli* but not S. *meliloti.* Alternatively, some other condition could be used such as minimal salts agar, which supports the growth of *S*. *meliloti* but not the growth of *E*. *coli.* Single colonies should appear within a week of incubation. Kanamycin (or Neomycin) is important because it allows the growth of only the transformants.

Confirmation of integration of ACIT at the chromosomal site of the gene of interest is performed by using colony PCR. Ideally, for the colony PCR, one primer should be specific to ACIT and a second primer specific to the chromosome. See Figure 5 for a map of ACIT along with the location of primers.

### Description of the drawings

- Fig. 1:: Fig. 1 schematically shows the widespread applicability of ACIT and a simple overview of the ACIT layout.
- Fig. 2:: Scheme of the native (upper panel) and the modified chromosome after insertion of ACIT, allowing control over the expression of the chromosomal copy of a gene of interest.
- Fig. 3:: Map of ACIT showing the location and orientation of the parts of ACIT, including the suicide vector pK18mob2 and the M13 primer used for sequencing: sequence *HindIII,* flanking modified 16S promoter: AAGCTT; sequence *Xbal,* flanking modified 16S promoter: TCTAGA; transcription terminator ("omega"), *Kpnl:* GGTACC; Sequence of *phaP* promoter, *SalI:* GTCGAC; Begin of *lacI: SacI*: GAGCTC; End of *lacI: EcoRI:* GAATTC.
- Fig. 4:: Nucleotide sequence and location of 16S promoter flanked by Lac operators, forming part 1 of ACIT: 
AAGCTTGGCAGTGAGCGCAACGCAATTAAATTGTTACGGAGCCCA
AAAAATCCGCTTGCGCCCGGGGCCGTCTGCTCCTAGAAACCGCTT
CACCGAGACATTGTGAGCGGATAACAATTCTAGA (SEQ ID no. 4).
- Fig. 5:: Scheme of ACIT designed for controlling expression of the gene of interest *dnaA.* Following integration into the chromosome, PCR analysis was used to confirm the location and origentation of ACIT in the chromosome.
- Fig. 6A:: Scheme of the native and the modified chromosome after insertion of ACIT, allowing control over the expression of the chromosomal copy of *phaC.*
- Fig. 6B:: Detection of fluorescence in cells after staining with Nile Red. WT = wild type. *phaC* promoterless is a control strain which has been modified to remove the promoter *of phaC from* the chromosomal copy of *phaC.* Error bars represent standard deviation from 3 biological replicates.
- Fig. 6C:: Merged photograph (bright field and fluorescence, 488 ex, 585 em) of the wild type (left) and the promoterless *phaC* strain (right). Bright spots within cells indicate PHB granules. Bars indicate 2 µm.
- Fig. 7:: Part A shows a scheme of the native (upper panel) and an exemplarily modified chromosome after insertion of ACIT_mraZ, allowing control over the expression of the *dcw* gene cluster. Part B shows that cells appear normal during exponential growth in the presence of IPTG (left panel), but exert massive elongation in the absence of IPTG (right panel). Bars indicate 2 µm.
- Fig. 8A:: Scheme of the native (upper panel) and the modified chromosome after insertion of ACIT_sinR, allowing control over the expression of *sinR.*
- Fig. 8B:: Quorum sensing mildly slows growth in S. *meliloti.* Higher levels of IPTG result in slower growth via the expression of *sinR.*
- Fig. 8C:: Expression of *sinR* has a strong impact on the expression of Sinl fused to mCherry. Error bars included for one curve are typical for all curves, and represent the standard deviation from 4 independent cultures; these are omitted from most curves to avoid cluttering the graphic.
- Fig. 8D:: Regulatory scheme outlining the role of SinR and Sinl within the quorum sensing regulation of galactoglucan production.
- Fig. 9A:: Scheme of the native (upper panel) and the modified chromosome after insertion of ACIT_expR, allowing control over the expression *of expR.*
- Fig. 9B:: Regulatory scheme outlining the role of ExpR within the quorum sensing regulation of galactoglucan production.
- Fig. 9C:: Galactoglucan production (opaque formation) on a solid agar culture upon addition of IPTG (broken lined ring indicates area of IPTG addition).
- Fig. 9D:: IPTG-induced expression of ExpR has a strong impact on the expression of *wgeA,* the first gene in the *wge* gene cluster.

## Claims

1. A transcription control cassette composed for the expression of at least one gene of interest, **characterised in that** the transcription control cassette comprises the following parts i) through vi),
i) a first DNA sequence comprising a modified 16S promoter DNA sequence having at least 70% nucleotide identity with SEQ ID no. 4, being flanked by a first Lac operator DNA sequence having at least 70% nucleotide identity with SEQ ID no. 1 and a second Lac operator DNA sequence having at least 70% nucleotide identity with SEQ ID no. 2;
ii) a second DNA sequence comprising a *lacl* gene-sequence having at least 70% nucleotide identity with SEQ ID no. 8;
iii) a third DNA sequence comprising a second nucleotide promotor sequence having a size in between 10 bp and 200 bp;
iv) a fourth DNA sequence comprising a transcription terminator sequence;
v) a fifth DNA sequence comprising a sequence conferring antibiotic resistance, acting as a marker gene;
vi) a sixth DNA sequence comprising a fragment from the gene of interest
having a size in between 300 to 1000 bp
which is ensuring the homologous recombination with the chromosomal sequence of the cell into which the transcription control cassette is to be transformed, whereat the fragment from the at least one gene of interest is comprising the first 300 to 1000 bp of the gene of interest;
whereat the DNA sequences of parts i) through vi) are separated from each other within the complete sequence of the transcription control cassette by DNA sequences of DNA restriction cleavage sites.

2. A transcription control cassette according to claim 1, whereat the second nucleotide promotor sequence according to part iii) is a DNA sequence having at least 70% nucleotide identity with SEQ ID no. 10 or a DNA sequence having at least 70% nucleotide identity with SEQ ID no. 11.

3. A transcription control cassette according to claim 1, whereat the fourth nucleic acid according to part iv) is a DNA sequence having at least 70% nucleotide identity with SEQ ID no. 9.

4. A transcription control cassette according to claim 1, whereat the sequence according to part v) is a DNA sequence having at least 70% nucleotide identity with SEQ ID no. 7.

5. A transcription control cassette according to claim 1, whereat the fragment from the at least one gene of interest according to part vi) is a DNA sequence having at least 70% nucleotide identity with SEQ ID no. 12, SEQ ID no. 12 being a fragment from the gene *sinR,* coding for the AHL synthase, or having at least 70% nucleotide identity with SEQ ID no. 13, SEQ ID no. 13 being a fragment from the gene *expR,* coding for an AHL receptor protein, or having at least 70% nucleotide identity with SEQ ID no. 14, SEQ ID no. 14 being a fragment from the gene *phaC,* coding for the synthase fo the production of polyhydroxybutyrate, or having at least 70% nucleotide identity with SEQ ID no. 15, SEQ ID no. 15 being a fragment from the gene *mraZ,* being the first gene in the *dcw* gene cluster, the *dcw* gene cluster being relevant for controlling cell division.

6. A transcription control cassette according to claim 1, **characterised in that** it comprises a DNA sequence having at least 70% nucleotide identity with SEQ ID no. 5, SEQ ID no. 5 comprising SEQ ID no. 14.

7. A transcription control cassette according to any one of preceeding claims 1 through 6, charcterised in that the expression of the nucleotide sequence of interest from the modified 16S promoter is inducible by the presence of a compound of initialisation.

8. A transcription control cassette according to claim 7, whereat the compound of initialisation for the transcription control cassette is IPTG.

9. A plasmide comprising a transcription control cassette according to any one of claims 1 through 8.

10. Usage of a transcription control cassette according to any one of claims 1 through 8 for the homologous or heterologous expression of at least one gene of interest in cells by transforming the transcription control cassette into these cells and cultivating them.

11. Usage according to claim 10, **characterised in that** the at least one gene of interest
- is expressed for the production of polyhydroxy butyric acid (PHB), being produced by the expression of the gene having at least 70% nucleotide identity with SEQ ID no. 16
or
- is expressed for the production of curdlan, whereat the production of curdlan requires at least the expression of the gene having at least 70% nucleotide identity with SEQ ID no. 17 and the gene having at least 70% nucleotide identity with SEQ ID no. 18 and the gene having at least 70% nucleotide identity with SEQ ID no. 19
or
- is expressed for the production of galactoglucan, being produced by the expression of the gene having at least 70% nucleotide identity with SEQ ID no. 20 and the other genes being located on the genomic map of *Sinorhizobium meliloti* from position 968095 until position 997228 of the megaplasmid pSymB, which are all together necessary for the production of galactoglucan.

12. A bacterial cell comprising the transcription control cassette of any one of claims 1 through 8 and the corresponding at least one gene of interest, whereat the at least one gene of interest is operatively linked to the transcription control cassette and both, the transcription control cassette and the at least one gene of interest, are located within the bacterial chromosome.

13. A bacterial cell according to claim 12, **characterised in that** it is a cell of a alphaproteobacteria.

14. A method for transformation of a bacterial cell with a transcription control cassette according to claim 1 by performing a biparental conjugation between a donator bacterium and the host bacterium, **characterised in that** it comprises the following steps,
a) a preparative step wherein the transcription control cassette according to claim 1 is used to transform the donator bacterium, providing a transformed donator bacterium;
b) a conjugation step wherein cells of the transformed donator bacterium from *step a)* and cells of the host bacterium which are to be transformed with the transcription control cassette according to claim 1 are
- harvested from separate cultures,
- resuspended in fresh liquid medium,
- mixed - preferredly at a ratio of 1:1 -,
- the mixture then being pipetted onto rich medium agar in spots and incubated, whereat incubation temperature is between 20°C and 45°C and incubation time is in between 4 hours and 3 days;
c) a selection step wherein a spot, being prepared and incubated according to step b), containing the bacterial mixture on agar
- is resuspended in an amount of liquid medium, then
- at least a part of this resuspension is plated on growth agar supplemented with a first antibiotic allowing selection for cells containing the transcription control cassette according to claim 1 and a second antibiotic
allowing selection for the cells of the host bacterium which are to be
transformed with the transcription control cassette according to claim 1,
or
- at least a part of this resuspension is plated on growth agar supplemented with an antibiotic allowing selection for cells containing the transcription control cassette according to claim 1, whereat the growth agar provides conditions which are preventing only the donator bacteria from growing, so that cells of the host bacterium containing the transcription control cassette according to claim 1 can grow,
then
- the growth agar being plated with at least a part of the resuspension is incubated until single colonies of the host bacterium, being transformed with a transcription control cassette according to claim 1, have been grown.
